# EUROPÄISCHE PATENTSCHRIFT

(11) **EP 1 880 695 B1**
(45) Veröffentlichungstag und Bekanntmachung des Hinweises auf die Patenterteilung: **09.09.2009**
(21) Anmeldenummer: 07014079.3
(22) Anmeldetag: 18.07.2007
(51) Int. Cl.: A61F 2/84, A61F 2/90, A61B 17/00

(54) **Röntgenmarker zur präzisen Implantation von Stents**
X-ray marker for precise implantation of stents
Marqueur de radiographie destiné à l'implantation précise de expanseurs

(30) Priorität: 19.07.2006 DE 102006033399
(43) Veröffentlichungstag der Anmeldung: 23.01.2008
(73) Patentinhaber: JOTEC GmbH, 72379 Hechingen (DE)
(72) Erfinder: Kaufmann, Ralf Dr., 72414 Rangendingen (DE)
(74) Vertreter: Laufer, Gabriele

(56) Entgegenhaltungen:
- EP-A- 1 466 570
- WO-A-00/71058
- WO-A-99/49812
- US-A1- 2004 204 749
- US-A1- 2006 100 688

## Beschreibung

Die vorliegende Erfindung betrifft ein Markerelement zum Markieren einer oder mehrerer Begrenzungen eines Abschnittes eines selbstexpandierenden, Maschen aufweisenden Stents, wobei das Markerelement röntgendichtes Material aufweist und sich auf einer Vorrichtung zum Einführen des Stents in ein Körpergefäß befindet, wobei die Vorrichtung ein röhrenförmiges Element aufweist, das durch den Stent hindurchgeführt ist.

Marker oder Markerelemente, die ein röntgendichtes Material aufweisen, werden bei der Einführung von Stents in Körpergefäße eines Patienten dazu eingesetzt, um die Position des Stents und/oder der Einführvorrichtung unter Röntgendurchleuchtung zu kontrollieren und die Einführung und Freisetzung genau zu verfolgen.

Stents sind Endoprothesen aus verschiedenen Materialien, die allgemein der Offenhaltung von Körpergefäßen dienen, und die röhrenartig und/oder als Maschengeflecht mit oder ohne Mantel ausgebildet sind. Stents werden in komprimierter Form über ein geeignetes Einführsystem in die Gefäße eingeführt und am Bestimmungsort zum dortigen Verbleib entfaltet. Dabei legen sich die Stentwände intraluminal an die Gefäßwände an. Selbstexpandierende Stents können bspw. für die Einführung in ein Gefäß durch einen Hüllschlauch komprimiert werden, der nach korrekter Platzierung zurückgezogen wird, so dass sich der selbstexpandierende Stent aufgrund seiner Elastizität entfalten und ausdehnen kann.

Im Stand der Technik ist bekannt, röntgendichte Marker an einem Einführsystem für Stents in ein Körpergefäß vorzusehen, die als ring- und röhrenförmige Strukturen an den entsprechenden Funktionselementen, Hüllschlauch, Drahtführungskatheter oder Pusher fest geklebt oder formschlüssig durch Formschmieden, dem so genannten "Swaging", angebracht sind. Es sind auch Marker an Einführsystemen bekannt, die als röntgendichte Substanzen in die Extrusions- oder Formmasse vor der Herstellung der Funktionselemente eingebracht werden.

All diese Marker dienen zur Überwachung der Position im Körper und zur Kontrolle der Funktion der Vorrichtungen während der Implantation des Stents.

Auch an den Stents selbst werden, wenn erforderlich, röntgendichte Marker befestigt. Der Stand der Technik kennt Marker, die in entsprechende Ösen lasergeschnittener Stents eingepresst, eingeschweißt, eingelötet oder eingeklebt sind. In der US 6,409,752 wird eine solche Befestigungsmethode gezeigt.

Die Marker dienen in der Regel zur Markierung der Stentenden unter Röntgendurchleuchtung. So können generell auch im mittleren Stentbereich Beginn oder Ende spezieller Stentzonen mit bestimmten mechanisch-physikalischen, chemischen oder biologischen Eigenschaften markiert werden, weil im zusammengefalteten Zustand praktisch keine Strukturen oder Übergänge an einem Stent erkennbar sind.

In der US 5,725,572 wird als weitere Möglichkeit zur Markierung der Stentenden ein röntgendichtes Material beschrieben, dass lokal als dünne Schicht auf den Stent aufgebracht ist. Dies kann durch gezieltes Bedampfen oder Abscheiden röntgendichter Substanzen an definierten Stellen des Stents geschehen. Die röntgendichten Marker sollen in allen Fällen, wie beabsichtigt, nicht von den Stents ablösbar sein.

Ferner wird in der WO 98/57692 ein expandierbarer Stent offenbart sowie ein Einführungssystem für diesen Stent in ein Körperlumen, das eine innere und eine äußere Röhre aufweist. Das in dieser internationalen Patentanmeldung offenbarte Einführungssystem weist u.a. röntgendichte ringförmige Marker auf, die fest auf dem äußeren und inneren Rohr angebracht sind, und zur Einklemmung des den Stent komprimierenden Hüllschlauchs dienen. Die beiden fest auf dem inneren Rohr angebrachten Marker zeigen dabei jeweils die Stentenden an. Durch ein Verschieben der Röhren gegeneinander wird der Hüllschlauch von dem Stent abgezogen und dieser freigesetzt.

Im Gegensatz zu lasergeschnittenen Stents bietet der beschriebene Stand der Technik bei selbstexpandierenden Stents, die aus einzelnen verflochtenen Drähten bestehen, so genannten Flechtstents, bei der lokalen, quasi punktförmigen Röntgenmarkierung keine oder nur unzulängliche Möglichkeiten zur Sichtbarmachung der Stentenden oder der Grenzen spezieller Stentzonen mit bestimmten mechanisch-physikalischen, chemischen oder biologischen Eigenschaften unter Röntgendurchleuchtung.

Ein solcher, aus Drähten geflochtener Stent, der eine spezielle, so genannte funktionelle Zone aufweist, die sich hier durch ihre besondere mechanisch-physikalische Eigenschaft auszeichnet, ist in der Patentanmeldung DE 103 35 649 beschrieben, auf die hiermit ausdrücklich Bezug genommen wird. Unter "funktioneller Zone" wird hierbei und im Folgenden ein Bereich des Stents verstanden, vorzugsweise in einem mittleren Abschnitt des Stents, der einen kleineren Flechtwinkel aufweist als in seinen sich in Längsrichtung an diesen Bereich anschließenden Abschnitten, vorzugsweise den proximalen und distalen Abschnitten.

Bei solchen Stents taucht das Problem auf, dass diese sich bei ihrer Freisetzung stark verkürzen. Dabei ist das Verhältnis 1/L der Stentlänge 1 im geladenen Zustand zur freien Stentlänge L von dem Innendurchmesser d des Hüllschlauches, dem Durchmesser D des Stents im entladenen Zustand sowie vom Flechtwinkel α abhängig. So wird beispielsweise ein Stent der Länge L = 40 mm, dem Durchmesser D = 6 mm und einem Flechtwinkel α = 40°, wenn er auf einen Durchmesser d = 1,5 mm komprimiert ist, um den Faktor 1,53 länger. Bei einem Stent mit funktioneller Zone, z. B. mit einem dortigen Flechtwinkel α = 10° kann die Verlängerung im Hüllschlauch des Einführsystems sogar um den Faktor 1/L = 4 bis 6 erfolgen.

Eine brauchbare, scharf abgegrenzte, bzw. quasi punktförmige Markierung inmitten eines Flechtstents zur präzisen Kennzeichnung bestimmter funktioneller Zonen, die für eine exakte Positionierung in einer Gefäßläsion erforderlich ist, ist bisher nicht bekannt. Im Stand der Technik ist bisher lediglich bekannt, röntgendichte Materialien an den Drahtenden durch Schweißen, Löten oder Kleben zu befestigen, wie bspw. durch die in der EP 0 858 299 beschriebenen Technik gezeigt.

Auch können hohlzylinderförmige Röntgenmarker vor dem Flechten inmitten des Stents auf den Flechtdraht aufgefädelt werden und dann angekrimpt oder angeklebt werden, aber diese Verfahren sind mit erheblichen Nachteilen behaftet.

So müssen bspw. die lokalen, quasi punktförmigen Marker neben der sehr hohen Röntgendichte auch ein ausreichend großes Volumen besitzen, um mit den derzeit üblichen Fluoroskopen deutlich erkennbar zu sein. Die aus extrem kleinen Drähten mit 0,05 mm bis 0,1 mm Durchmesser teilweise sehr dicht geflochtenen Stents, die zum Beispiel auch für den Einsatz in hirnversorgenden und intrakraniellen Blutgefäßen vorgesehen sind, bieten nicht genug Verankerungsmöglichkeiten für einen ausreichend erkennbaren, voluminösen Röntgenmarker.

Die quasi punktförmigen Marker können darüber hinaus oft erst nach der Herstellung und Oberflächenveredelung des Stents angebracht werden, da sie sonst aufgrund ihrer anderen chemischen und mechanischen Eigenschaften die Veredelung des Stents, zum Beispiel durch thermomechanische Prozesse und Oberflächenpolierverfahren, negativ beeinflussen.

Die elektrochemische Wechselwirkung zwischen dem Stentmaterial und einem lokal konzentrierten Markermaterial in Blut oder anderen Körperflüssigkeiten kann zur Auflösung der Verbindung und Korrosion des Stents selbst führen. Das so erzeugte Risiko eines embolischen Hirnschlages durch abkorrodierte Stent- und Markerteile ist nicht tragbar.

Der quasi punktförmige Marker muss auch die großen Formveränderungen der Flechtstruktur eines Stents während seiner Freisetzung, insbesondere die weiter oben beschriebene Verkürzung auf bis zu einem Sechstel seiner Länge aushalten und darf darüber hinaus nicht die Innenwand der filigranen Einführsysteme, die Innendurchmesser von 0,9 mm bis 1,8 mm aufweisen können, beschädigen. Durch einen solchen Abrieb erzeugte Partikel des Einführsystems können ebenfalls einen embolischen Hirnschlag beim Patienten hervorrufen.

Schließlich setzen Montage- und Verbindungstechniken wie Kleben, Löten, Schwei-ßen oder Krimpen quasi punktförmiger Marker im mittleren Bereich von geflochtenen Stents die Entwicklung neuer und unter Umständen aufwändiger Produktionsverfahren voraus.

Ein weiterer Marker ist aus der US-A-2004/0204749 vorbekannt.

Die der Erfindung zugrunde liegende Aufgabe ist es daher, eine oder beide Begrenzungen eines Abschnitts eines Maschen aufweisenden, selbstexpandierenden Stents, im Einführsystem und während seiner Freisetzung, in einfacher und sicherer Weise klar und deutlich unter Röntgendurchleuchtung kenntlich zu machen.

Diese Aufgabe wird erfindungsgemäß gemäß Ansprüchen 1 und 12 dadurch gelöst, dass das Markerelement zwischen Stent und röhrenförmigem Element verschiebbar auf dem röhrenförmigen Element angebracht ist, und in die Maschen des Stents ablösbar eingreift.

Die der Erfindung zugrunde liegende Aufgabe wird auf diese Weise vollkommen gelöst.

Mit dem erfindungsgemäßen Markerelement ist es möglich, auch Abschnitte eines Stents zu markieren, die sich während der Freisetzung des Stents während seiner Positionierung und Freisetzung bspw. in ihrer Länge verändern. Dies wird dadurch erreicht, dass das Markerelement mit dem in das Einführsystem geladenen Stent formschlüssig, aber ablösbar verbunden ist, bzw. ablösbar in die Maschen des Stents eingreift, wenn dieser sich im komprimierten Zustand, d.h. geladen auf dem Einführsystem, befindet.

Gleichzeitig ist das Markerelement hin und her verschiebbar, aber nicht ablösbar, auf dem Drahtführungskatheter gelagert, und zwar zwischen diesem und dem Stent. Das Markerelement kann dabei an einer Stelle mit dem Stent in ablösbarer Weise verbunden sein, wo es bspw. den Beginn oder das Ende einer Stentzone mit speziellen mechanisch-physikalischen, chemischen oder biologischen Eigenschaften unter Röntgendurchleuchtung markieren soll.

Unter "selbstexpandierender Stent" wird jeder Stent oder Stent-Graft verstanden, der ein Gerüst, bspw. ein Drahtgerüst, aus einem selbstexpandierenden Material aufweist, wobei das Gerüst darüber hinaus mit einem Textilschlauch verbunden sein kann; zur Implantation in ein Körpergefäß ist dabei der Stent radial zusammendrückbar, bspw. durch einen Hüllschlauch, so dass sich seine Querschnittsfläche deutlich verringert und er einfach in das Gefäß eingeführt werden kann; wird die Kompression entlastet, also bspw. der Hüllschlauch entfernt, so entfaltet sich der Stent aufgrund der Federwirkung seines Rahmens wieder radial in seine ursprüngliche Form, die sich innen in dem Körpergefäß verklemmt.

Unter "eine oder mehrere Begrenzungen eines Abschnittes eines selbstexpandierenden Stents" kann dabei einerseits das eine oder andere Ende des Stents gemeint sein, d.h. der Abschnitt wird in diesem Falle als der gesamte Stent definiert. Andererseits kann aber auch ein bestimmter Abschnitt innerhalb des Stents gemeint sein, der sich aufgrund unterschiedlicher mechanisch-physikalischer, chemischer oder biologischer Eigenschaften von den anderen Abschnitten des Stents unterscheidet. Dies kann bspw. eine so genannte funktionelle Zone eines Flechtstents sein, wie sie bspw. in der DE 103 35 649 offenbart und definiert ist. Hier ist ein Flechtstent offenbart, der aus einem Geflecht aus einer Vielzahl von fadenförmigen Elementen besteht, die im expandierten Zustand des Flechtstents eine Ebene senkrecht zur Längsrichtung unter einem Flechtwinkel schneiden. Der Stent weist dabei eine Zone auf (im folgenden "funktionelle Zone"), die einen kleineren Flechtwinkel aufweist als die sich an diese Zone angrenzenden Abschnitte oder Zonen des Flechtstents. In diesem Zusammenhang bedeutet also bspw. "eine Begrenzung eines Abschnittes" die Begrenzung der funktionellen Zone.

"Röntgendichtes Material" soll vorliegend jedes Material bedeuten, das für Röntgenstrahlen nicht oder nur gering durchlässig ist, und das daher bei Einsatz entsprechender Bild gebender Verfahren, wie bspw. der Röntgen-Durchstrahlung (Fluoroskopie), sichtbar ist, bspw. als Fleck. Mit dem erfindungegemäßen Markerelement werden die Nachteile des Stande der Technik umgangen, und eine fluoroskopisch klar und deutlich sichtbare Markierung einer oder beider Begrenzungen eines Abschnitts eines selbstexpandierenden, Maschen aufweisenden Stents während seiner Positionierung und Freisetzung in ein Körpergefäß ermöglicht.

Das Markerelement kann dabei insgesamt einstückig ausgebildet sein, oder aber mehrere Bestandteilen aufweisen, die miteinander verbunden sind.

Das Markerelement, das eine ausreichende Masse an röntgendichtem Material enthält, ist im Inneren des geladenen Stents am Ort der zu markierenden Begrenzung formschlüssig mit dem Stent verankert.

In einer Ausführungsform ist dabei bevorzugt, wenn das röhrenförmige Element ein Drahtführungskatheter ist. Hierbei ist also der Stent zur Einführung in ein Körpergefäß auf eine Vorrichtung zum Einführen des Stents geladen, das u.a. einen Drahtführungskatheter aufweist, und das Markerelement ist zwischen Stent und Drahtführungskatheter verschiebbar auf dem Drahtführungskatheter gelagert, wobei das Markerelement in die Maschen des Stents eingreift.

Wenn sich der Stent also während seiner Freisetzung innerhalb des Einführsystems bewegt, nimmt er das Markerelement in entsprechender Position mit. Erst wenn der markierte Stentbereich den Hüllschlauch verlässt und sich der Stent dort aufgrund seines selbstexpandierenden Mechanismus vom Einführsystem ablöst, löst er sich gleichzeitig auch vom auf dem Drahtführungskatheter angebrachten Markerelement ab, das verschiebbar, aber nicht ablösbar mit diesem verbunden ist.

Der Marker steht also während der Positionierung und Implantation des Stents wie ein Teil des Stents selbst zur Verfügung, wird aber nach der Freisetzung des Stents als Teil der Vorrichtung mit dieser aus dem Körper entfernt. Durch diese Konstruktion können quasi punktförmige Marker mit einer sehr hohen Röntgendichte und einem ausreichend großen Volumen am Stent realisiert werden.

Die aus extrem kleinen Drähten mit 0,05 mm bis 0,1 mm Durchmesser und sehr dicht geflochtenen Stents werden nicht mehr als ohnehin unzulängliche Verankerungsmöglichkeiten für relativ voluminöse Röntgenmarker benötigt.

Bei der Herstellung und Veredelung der Stents müssen keine komplexen chemischen und mechanischen Wechselwirkungen mit dem röntgendichten Material durch thermomechanische Prozesse und Oberflächenpolierverfahren in Kauf genommen werden.

Die elektrochemische Wechselwirkung zwischen Stentmaterial und lokal konzentriertem Markermaterial und das so erzeugte Risiko eines embolischen Hirnschlages durch abkorrodierte Stent- und Markerteile wird umgangen.

Der quasi punktförmige Marker kann die großen Formveränderungen der Flechtstruktur des Stents während seiner Freisetzung, insbesondere die im Extremfall vorhandene Verkürzung auf bis zu einem Sechstel seiner Länge im geladenen Zustand aushalten und kann durch seine Lage im Inneren des zusammengefalteten Stents nicht die Innenwand der filigranen Vorrichtungen bzw. Einführsysteme beschädigen.

Dabei ist von Vorteil, dass das Markerelement durch seinen Formschluss mit dem zusammengedrückten Stent den Beginn oder das Ende einer Funktionellen Zone des Stents markiert, auch wenn diese sich während des Freisetzens des Stents axial gegenüber dem Drahtführungskatheter verschiebt.

Für Nachuntersuchungen, bei denen in der Regel Einzelbilder durch stationäre leistungsfähigere Röntgengeräte erstellt werden, sind die wesentlich dichteren Geflechte der hier beschriebenen funktionellen Zonen ausreichend abgrenzbar.

Bei einer weiteren Ausführungsform ist bevorzugt, wenn das Markerelement ein hohlzylindrisches Element aufweist, das wiederum mindestens einen, vorzugsweise 1 bis 12 radial nach außen weisenden Vorsprung aufweist, der in die Maschen des Stents eingreift.

Diese Ausführungsform hat den Vorteil, dass mit dem oder den Vorsprüngen eine Art Ankerelement oder Ankerelemente bereitgestellt werden, die in den Stent eingreifen und sich in diesem verankern.

Dabei kann vorgesehen sein, dass die Vorsprünge prismen- oder pyramidenförmig ausgebildet sind. Im Falle einer Ausbildung der Vorsprünge/Anker als prismatische Körper ist bevorzugt, wenn diese mit der Stirnfläche zum Hohlzylinderkörper hin ausgeführt sind. Im Falle der Ausbildung der Vorsprünge/Anker als Pyramidenstümpfe ist bevorzugt, wenn diese mit der großen Stirnfläche zum Hohlzylindrischen Körper hin ausgeführt sind.

Insbesondere ist bevorzugt, wenn die Höhen der Vorsprünge/Anker so bemessen sind, dass das Markerelement genügend Spiel zum Hüllschlauch hat, die Höhen aber kleiner sind als die Wand- oder Flechtdrahtstärke des Stens, so dass der Stent im zusammengedrückten Zustand nicht vom Markerelement abgleiten kann.

Die Erfindung betrifft ferner eine Vorrichtung zum Einführen eines selbstexpandierenden Stents in ein Körpergefäß, wobei die Vorrichtung Folgendes aufweist:
- einen Hüllschlauch, der einen Stent in einem distalen Abschnitt des Einführsystems radial zusammengedrückt hält,
- einen im Hüllschlauch geführten Pusher, der mit seinem distalen Ende an das proximale Ende des Stents stößt,
- ein röhrenförmiges Element, vorzugsweise ein Drahtführungskatheter, das durch den zusammengedrückten Stent und den Pusher hindurch geführt ist,
wobei sie ferner ein Markerelement aufweist, das frei hin und her verschiebbar, aber nicht ablösbar, auf dem röhrenförmigen Element angebracht ist, und das in ablösbarer Weise mit dem radial zusammengedrückten Stent verbunden ist.

Insbesondere ist bevorzugt, wenn die Vorrichtung ein Markerelement wie weiter oben aufgeführt aufweist.

In einer weiteren Ausführungsform ist bevorzugt, wenn bei der erfindungsgemäßen Vorrichtung ein Hüllschlauch vorgesehen ist, der einen Hüllschlauchmarker aufweist. Es versteht sich, dass das Markerelement auf jedem röhren- oder kegelförmigen Element verschiebbar angebracht werden kann, auf welches ein Stent zur Einbringung in ein Körpergefäß geladen wird. Wichtig für die vorliegende Erfindung ist, dass das Markerelement in den Stent auf einen ablösbare Art und Weise eingreifen kann, wobei es gleichzeitig verschiebbar, aber auf einen nicht-ablösbare Weise auf dem röhrenförmigen Element angebracht ist, und sich somit zwischen Stent und röhrenförmigem Element befindet.

Als röntgendichtes Material kann vorliegend jedes im Stand der Technik bekannte röntgendichte Material eingesetzt werden, das sich als geeignet für die vorliegenden Zwecke erwiesen hat. Geeignet sind dabei bspw. schwere und biokompatible Metalle, wie bspw. die Edelmetalle Gold, Platin, Iridium, Platin-Iridium, aber auch Metalle wie Tantal, wobei dem Fachmann klar sein wird, dass bei dem erfindungsgemäßen Markerelement auch andere, hier nicht aufgeführt Materialien zum Einsatz kommen können.

Bei der erfindungsgemäßen Vorrichtung, also dem Einführsystem, ist ein Hüllschlauch vorgesehen, der den Stent radial zusammengedrückt hält. Wenn der Hüllschlauch nach proximal zurückgezogen wird, gibt er den Stent von distal nach proximal frei, so dass dieser sich aufgrund seines selbstexpandierenden Mechanismus von der Vorrichtung und auch von dem dort verschiebbar befestigten Markerelement ablöst. Das Einführsystem wird nach dem Absetzten des Stents mitsamt dem daran verschiebbar befestigten Markerelement aus dem Körpergefäß entfernt.

Mit dem Begriff "distal" wird vorliegend diejenige Richtung/dasjenige Ende der Vorrichtung oder Teile der Vorrichtung bezeichnet, die vom Anwender weg (also in Richtung Spitze des Einführsystems) führt; mit dem Ausdruck "proximal" wird diejenige Richtung/dasjenige Ende der Vorrichtung oder Teile der Vorrichtung bezeichnet, die zum Anwender hinführt; bzw. hinweist.

Ein Ausführungsbeispiel der Erfindung ist in den Zeichnungen dargestellt und wird in der nachfolgenden Beschreibung näher erläutert. Es zeigen:
- Fig. 1: eine schematische Darstellung einer Ausführungsform eines erfindungsgemäßen Einführsystem mit geladenem Stent, wobei die Darstellung nicht maßstabsgetreu sein soll, wobei der Hüllschlauch transparent ist und der Stent nur gestrichelt und seine funktionelle Zone nur gepunktet angedeutet ist;
- Fig. 2: einen Ausschnitt einer vergrößerten, schematischen und nicht maßstabsgetreuen Ansicht einer Ausführungsform des erfindungsgemäßen Markerelements im Einführsystem aus Fig. 1, wobei hier nur der Hüllschlauch längs geschnitten ist;
- Fig. 3: eine vergrößerte, schematische und nicht maßstabsgetreue Ansicht eines Längsschnitts durch das Markerelement aus Fig. 2;
- Fig. 4: eine schematische Darstellung des distalen Teils des Einführsystems aus Fig. 1 mit dem teilweise entladenen Stent, wobei bereits die distale Hälfte der funktionellen Zone des Stents freigesetzt ist;
- Fig. 5: eine schematische Darstellung des distalen Teils des Einführsystems aus Fig. 1 mit dem teilweise entladenen Stent, wobei gezeigt ist, wie das proximale Ende der funktionellen Zone des Stents austritt; und
- Fig. 6.: eine schematische Darstellung des distalen Teils des Einführsystems aus Fig. 1 mit dem vollständig entladenen Stent.

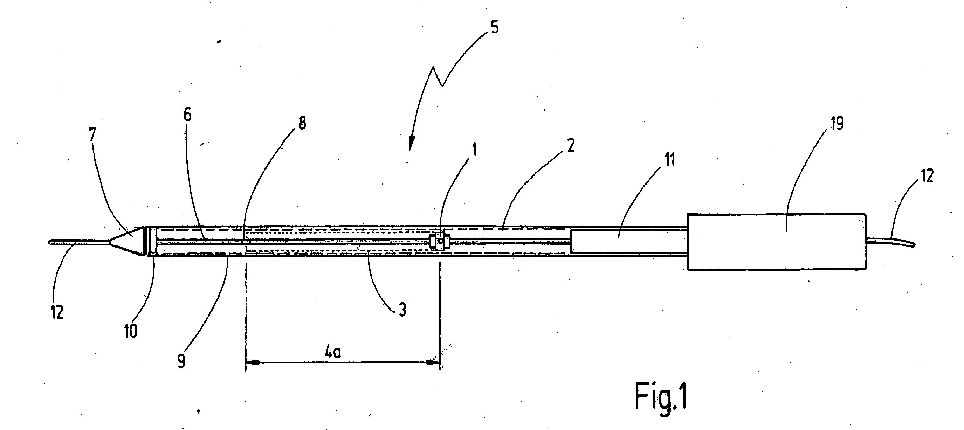

In Fig. 1 ist mit dem Bezugszeichen 5 insgesamt - schematisch - das Einführsystem bzw. die Vorrichtung zum Einführen des Stents bezeichnet, mit dem ein mit 2 angedeuteter Stent, der über eine mit 3 angedeutete funktionelle Zone verfügt, in ein Körpergefäß, bspw. ein Blutgefäß, eingeführt werden kann.

Der Stent 2 kann dabei ein selbstexpandierender Metallstent sein, der in Leinwandbindungstechnik hergestellt ist, wie dies in der eingangs erwähnten DE 103 35 649 beschrieben ist. Das Einführsystem 5 weist einen Griff 19 auf, der mit einem Hüllschlauch 9, einem Pusher 11 und einem Drahtführungskatheter 6 funktionell verbunden ist. Der Hüllschlauch 9 hält den Stent 2 radial zusammengedrückt. Der Hüllschlauch 9 weist an seinem distalen Ende einen Hüllschlauchmarker 10 aus röntgendichtem Material auf.

In diesem Zustand wird das Einführsystem 5 entsprechend der bekannten Seldinger-Methode über einen Führungsdraht 12 in ein Gefäß eingebracht. Dabei verläuft der Führungsdraht 12 durch die atraumatische Spitze 7 und den im Inneren des Stents 2 und des Einführsystems 5 liegenden Drahtführungskatheter 6 hindurch. Der Drahtführungskatheter 6 ist auch, hier nicht sichtbar, durch den Pusher 11 hindurch geführt.

Auf dem Drahtführungskatheter 6 ist ein Drahtführungskathetermarker 8 mit den eingangs erwähnten Methoden befestigt. Auf dem Drahtführungskatheter 6 befindet sich auch, was beim vorliegenden Stent sehr sinnvoll ist, das erfindungsgemäße verschiebbare Markerelement 1.

Das verschiebbare Markerelement 1 und der feste Drahtführungskathetermarker 8 begrenzen die funktionelle Zone 3 des Stents 2 im Einführsystem 5. In einer weiteren Ausführungsform, die hier nicht dargestellt ist, kann auch der distale Begrenzungsmarker wie das Markerelement 1 verschiebbar auf dem Drahtführungskatheter 6 angeordnet sein.

Mit 4a ist die Länge der funktionellen Zone 3 des Stents 2 im Einführsystem 5 beschrieben. Wenn das Einführsystem 5, beziehungsweise der zusammengefaltete Stent 2, beziehungsweise die funktionelle Zone 3 in der Zielgefäßläsion positioniert sind, wird der Stent 2 durch Ziehen des Hüllschlauches 6 nach proximal und durch gleichzeitiges Drücken des Pushers 11 nach distal freigesetzt.

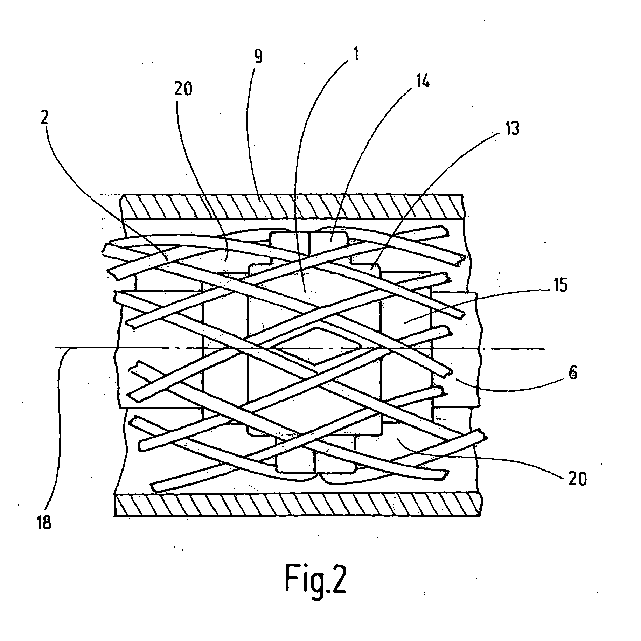

In Fig. 2 ist ein Ausführungsbeispiel des Markerelements 1 in einer vergrößerten, schematischen und nicht maßstabsgetreuen Ansicht im Einführsystem 5 dargestellt, wobei nur der Hüllschlauch 9 längs geschnitten ist. Das Markerelement 1 befindet sich innerhalb des zusammengedrückten Stents 2, der Maschen 20 aufweist, und ist wie ein Ring auf den Drahtführungskatheter 6 aufgefädelt.

Das Markerelement 1 besteht aus einem Hohlzylinderkörper 13, an dem sich radial nach außen hin zeigend, je nach Ausführungsform ein bis zwölf Vorsprünge/Anker 14 befinden können. In den Hohlzylinderkörper 13 ist eine Hülse 15 aus einem röntgendichten Material eingeschoben und fest mit dem Hohlzylinderkörper 13 des Markerelements 1 verbunden.

Bei einem geflochtenen Stent aus 24 Drähten ist zum Beispiel eine Ausführungsform des Markerelements 1 mit drei im 120° Winkel angeordneten Vorsprüngen/Ankern 14 zu bevorzugen. Je nach Anzahl der Flechtdrähte oder Art des Stents 2, geflochten oder lasergeschnitten, können andere Zahlen oder auch entlang der Mittelachse 18 des Hohlzylinderkörpers 13 versetzte Anordnungen für die Vorsprünge/Anker 14 gewählt werden.

Die Vorsprünge/Anker 14 durchdringen die Maschen 20 oder das Geflecht des zusammengefalteten Stents 2. Die Vorsprünge/Anker 14 sind vorzugsweise als Prismen oder Pyramidenstümpfe ohne Hinterschnitt auszuführen, damit sich der Stent 2, wenn er während seiner Freisetzung radial expandiert, leicht vom Markerelement 1 ablöst.

Der Hohlzylinderkörper 13 mit dem oder den Vorsprüngen/Ankern 14 ist vorzugsweise aus einem medizinisch geeigneten Kunststoff, wie zum Beispiel Polyoxymethylen (POM), im Kunststoffspritzgussverfahren hergestellt. Die Hülse 15 ist vorzugsweise aus einem sehr röntgendichten Metall oder einer Legierung, wie zum Beispiel Gold, Platin, Tantal oder einer Platin-Iridium-Legierung, gefertigt.

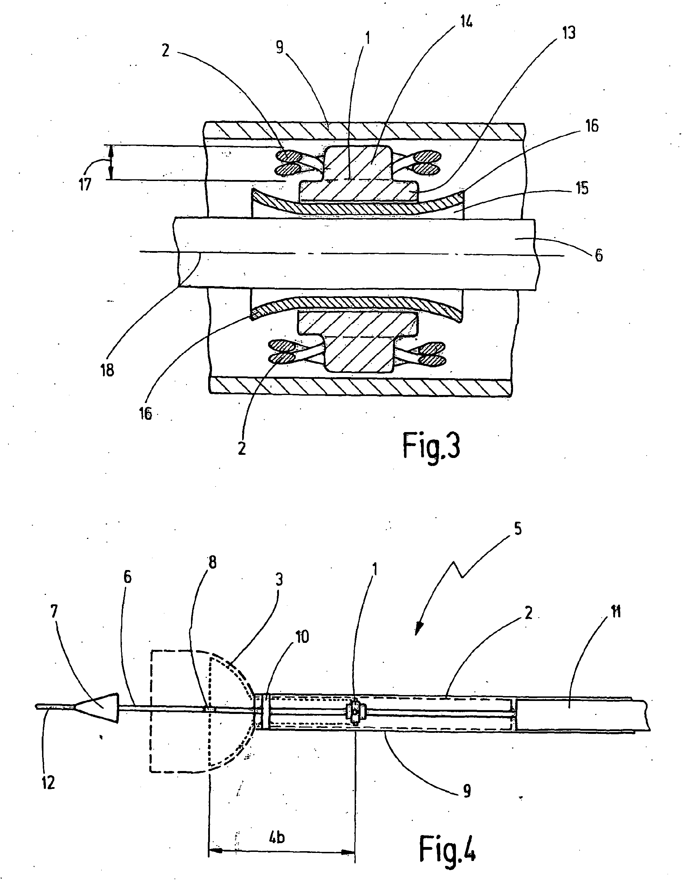

Fig. 3 zeigt einen vergrößerten, schematischen und nicht maßstabsgetreuen Längsschnitt durch das Markerelement aus Fig. 2. In der gezeigten Ausführungsform des Markerelements 1 ist die Hülse 15 durch beidseitige Aufweitungen 16 formschlüssig mit dem Hohlzylinderkörper 13 verbunden. Dadurch ist sichergestellt, dass sich die Hülse 15 nicht vom Hohlzylinderkörper 13 in Richtung der Mittelachse 18 ablösen kann.

Eine Presspassung zwischen Hülse 15 und Hohlzylinderkörper 13 wäre in einer weiteren Ausführungsform denkbar, aber dies könnte zu einer Überlastung und einem Bruch des Hohlzylinderkörpers 13 durch spannungsinduzierte mechanische Kriechprozesse führen. Die Hülse 15 könnte sich dann in Richtung der Mittelachse 18 ablösen. Zwischen Hülse 15 und Drahtführungskatheter 6 muss ausreichend Spiel sein, damit diese, bzw. das Markerelement 1 leicht auf dem Drahtführungskatheter 6 gleiten kann.

Die Höhe 17 der Vorsprünge/Anker 14 ist so hoch bemessen, dass der zusammengefaltete Stent 2 nicht zwischen Hüllschlauch 9 und Vorsprung/Anker 14 in Richtung der Mittelachse 18 vom Markerelement 1 abgleiten kann. Die Höhe 17 der Vorsprung/Anker 14 muss aber so gering bemessen sein, dass das Markerelement 1 nicht im Hüllschlauch 9 verklemmen kann.

Eine Aufweitung 16 der Hülse 15 sorgt während des Gleitens des dann noch mit dem Stent 2 gekoppelten Markerelements 1 über dem Drahtführungskatheter 6 beim Freisetzen des Stents 2 dafür, dass es zu keinem Verklemmen und Verkanten des Markerelements 1 mit dem Drahtführungskatheter 6 kommt.

In den Fig. 4 bis 6 ist der Ablauf der Freisetzung eines Stents 2 am distalen Teil des Einführsystems 5 aus Fig. 1 gezeigt. In Fig. 4 ist ein Teil des Stents 2 freigesetzt. Die distale Hälfte seiner funktionellen Zone 3 hat, unter Röntgendurchleuchtung sichtbar, den Hüllschlauchmarker 10 passiert. Das Markerelement 1 hat inzwischen relativ zum Drahtführungskatheter 6 eine Bewegung nach distal vollführt. Die Länge 4b der funktionellen Zone 3 des teilweise entladenen Stents 2 ist also kürzer als die Länge 4a, wie sie im Anfangszustand, der in Fig. 1 dargestellt ist. Der Stent 2 und somit auch seine funktionelle Zone 3 sind bereits aufgrund ihrer Struktur kontrahiert.

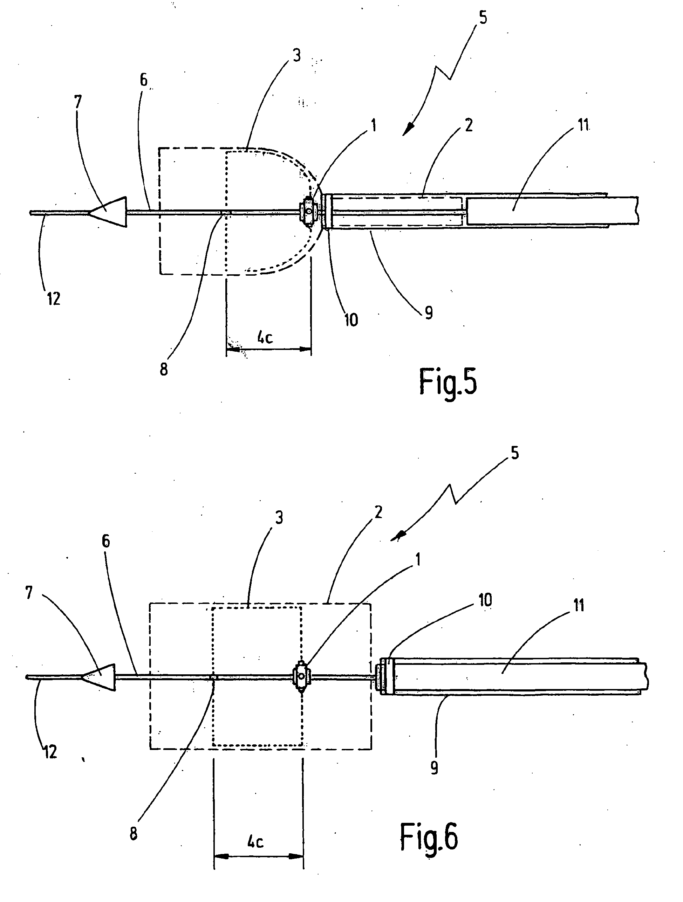

In Fig. 5 ist ein noch größerer Teil des Stents 2 entladen, bzw. freigesetzt, dargestellt. Das proximale Ende seiner funktionellen Zone 3 hat gerade den Hüllschlauchmarker 10 passiert. Der Stent 2 hat sich weiter verkürzt. Somit hat sich auch die Länge 4c der funktionellen Zone 3 gegenüber der Länge 4b aus Fig. 4 weiter verkürzt. Diese Verkürzung kann, wie bereits erwähnt, bei geflochtenen Stents bis zu einem sechstel der Länge im zusammengefalteten Zustand betragen. Das Markerelement 1 hat sich also aufgrund seiner formschlüssigen Verbindung mit dem Stent 2 durch seine Anker 14 relativ zum Drahtführungskatheter 6 weiter nach distal bewegt.

In Fig. 6 ist der distale Teil des Einführsystems 5 aus Fig. 1 mit dem vollständig entladenen Stent 2 dargestellt. Der Stent 2 hat sich in diesem Zustand vom Markerelement 1 nach radial außen abgelöst. Der Stent 2 hat sich entsprechend seiner Struktur verkürzt. Die Länge 4c der funktionellen Zone 3 des Stents 2 und die Position des nun vom Stent 2 abgelösten Markerelements 1 bleiben konstant.

Das Einführsystem 5 wird nach dem Absetzten des Stents 2 mitsamt des daran verschiebbar befestigten Markerelements 1 aus dem Körpergefäß entfernt.

### Bezugszeichenliste

- 1: Marker
- 2: Stent
- 3: funktionelle Zone (des Stents 2)
- 4a: Länge (der funktionellen Zone 3 des Stents 2 im Einführsystem 5)
- 4b: Länge (der funktionellen Zone 3 des teilweise entladenen Stents 2)
- 4c: Länge (der vollständig freigesetzten funktionellen Zone 3)
- 5: Einführsystem
- 6: Drahtführungskatheter/röhrenförmiges Element
- 7: Spitze
- 8: Drahtführungskathetermarker
- 9: Hüllschlauch
- 10: Hüllschlauchmarker
- 11: Pusher
- 12: Führungsdraht
- 13: Hohlzylinderkörper
- 14: Anker
- 15: Hülse
- 16: Aufweitung
- 17: Höhe (der Anker 14)
- 18: Mittelachse
- 19: Griff
- 20: Masche

## Patentansprüche

1. Markerelement (1) zum Markieren einer oder mehrerer Begrenzungen eines Abschnittes eines selbstexpandierenden, Maschen (20) aufweisenden Stents (2), wobei das Markerelement (1) ein röntgendichtes Material aufweist und auf einer Vorrichtung (5) zum Einführen des selbstexpandierenden Stents (2) in ein Körpergefäß anbringbar ist, wobei die Vorrichtung (5) ein röhrenförmiges Element (6) aufweist, das durch den Stent (2) hindurchgeführt ist, und wobei das Markerelement (1) ablösbar in die Maschen (20) des Stents (2) eingreift, **dadurch gekennzeichnet, dass** das Markerelement (1) zwischen Stent (2) und röhrenförmigem Element (6) verschiebbar auf dem röhrenförmigen Element (6) anbringbar ist.

2. Markerelement (1) nach Anspruch 1, **dadurch gekennzeichnet, dass** das röhrenförmige Element ein Drahtführungskatheter (6) ist.

3. Markerelement (1) nach Anspruch 1 oder 2, **dadurch gekennzeichnet, dass** es sich bei der Freisetzung und Expandierung des Stents (2) vom Stent (2) ablöst.

4. Markerelement (1) nach einem der Ansprüche 1 bis 3, **dadurch gekennzeichnet, dass** es ein Ende einer funktionellen Zone (3) des Stent (2) markiert, wobei die funktionelle Zone (3) einen Abschnitt des Stents (2) darstellt, der einen kleineren Flechtwinkel aufweist als die sich in Längsrichtung anschließenden weiteren Abschnitte des Stents (2).

5. Markerelement (1) nach einem der Ansprüche 1 bis 4, **dadurch gekennzeichnet, dass** es ein Hohlzylinderförmiges Element (13) aufweist, das wiederum mindestens einen, vorzugsweise 1 bis 12, radial nach außen weisenden Vorsprung (14) aufweist, der in die Maschen (20) des Stents (2) eingreift.

6. Markerelement (1) nach Anspruch 5, **dadurch gekennzeichnet, dass** der zumindest eine Vorsprung (14) in Form eines Prismas ausgebildet ist.

7. Markerelement (1) nach Anspruch 5, **dadurch gekennzeichnet, dass** der zumindest eine Vorsprung (14) pyramidenförmig ist.

8. Markerelement (1) nach einem der Ansprüche 1 bis 7, **dadurch gekennzeichnet, dass** es vollständig aus röntgendichtem Material besteht.

9. Markerelement (1) nach einem der Ansprüche 5 bis 7, **dadurch gekennzeichnet, dass** es ferner einen Hülsenkörper (15) aufweist, das in das Hohlzylinderförmige Element (13) eingeführt und fest mit diesem verbunden ist.

10. Markerelement (1) nach Anspruch 9, **dadurch gekennzeichnet, dass** der Hülsenkörper (15) ein röntgendichtes Material aufweist, und das Hohlzylinderförmige Element (13) ein nicht-röntgendichtes Material.

11. Marker nach Anspruch 9 oder 10, **dadurch gekennzeichnet, dass** der Hülsenkörper (15) an seinen umlaufenden Enden Aufweitungen (16) aufweist, um zu verhindern, dass sich das Hohlzylinderförmige Element (13), in das der Hülsenkörper (15) eingeführt ist, von dem Hülsenkörper (15) ablöst.

12. Vorrichtung zum Einführen (5) eines selbstexpandierenden Stents (2) in ein Körpergefäß, aufweisend
- einen Hüllschlauch (9), der einen Stent (2) in einem distalen Abschnitt des Einführsystems (5) radial zusammengedrückt hält,
- einen im Hüllschlauch (9) geführten Pusher (11), der mit seinem distalen Ende an das proximale Ende des Stents (2) stößt,
- ein röhrenförmiges Element (6), das durch den zusammengedrückten Stent (2) und den Pusher (11) hindurch geführt ist,
- **dadurch gekennzeichnet ist, dass** es ferner ein Markerelement (1) aufweist, das frei hin und her verschiebbar, aber nicht ablösbar, auf dem röhrenförmigen Element (6) angebracht ist, und das in ablösbarer Weise mit dem radial zusammengedrückten Stent (2) verbunden ist.

13. Einführsystem (5) nach Anspruch 12, **dadurch gekennzeichnet, dass** der Hüllschlauch (9) einen Hüllschlauchmarker (10) an seinem distalen Ende aufweist.

14. Einführsystem (5) nach Anspruch 12 oder 13, **dadurch gekennzeichnet, dass** das röhrenförmige Element ein Drahtführungskatheter (6) ist.

15. Einführsystem (5) nach einem der Ansprüche 12 bis 14, **dadurch gekennzeichnet, dass** es ein Markelement nach einem der Ansprüche 1 bis 10 aufweist.

## Claims

1. Marker element (1) for marking one or more boundaries of a portion of a self-expanding stent (2), the stent(2) comprising meshes (20), and the marker element (1) comprising a radiopaque material and being fittable on a device (5) for the delivery of the self-expanding stent (2) into a body vessel, the device (5) comprising a tubular element (6) which is guided through the stent (2), wherein the marker element (1) detachably engages with the meshes (20) of the stent (2), **characterized in that** the marker element (1) between the stent (2) and the tubular element (6) is slidably fittable on the tubular element (6.

2. Marker element (1) according to Claim 1, **characterized in that** the tubular element is a guide wire catheter (6).

3. Marker element (1) according to Claim 1 or 2, **characterized in that** it detaches from the stent (2) during deployment and expansion of the stent (2).

4. Marker element (1) according to one of Claims 1 to 3, **characterized in that** it marks an end of a functional zone (3) of the stent (2), the functional zone (3) representing a portion of the stent (2) with a lower braiding angle than the portions of the stent (2) adjoining in longitudinal direction.

5. Marker element (1) according to one of Claims 1 to 4, **characterized in that** it has a hollow cylindrical element (13) which in turn comprises at least one, preferably 1 to 12, radially outward-pointing projection (14) which engages into the meshes (20) of the stent (2).

6. Marker element (1) according to Claim 5, **characterized in that** the at least one projection (14) has the form of a prism.

7. Marker element (1) according to Claim 5, **characterized in that** the at least one projection (14) has the form of a pyramid.

8. Marker element (1) according to one of Claims 1 to 7, **characterized in that** it is made completely of radiopaque material.

9. Marker element (1) according to one of Claims 5 to 7, **characterized in that** it also has a sleeve body (15) which fits inside the hollow cylindrical element (13) and is firmly connected to it.

10. Marker element (1) according to Claim 9, **characterized in that** the sleeve body (15) comprises a radiopaque material and the hollow cylindrical element (13) a non-radiopaque material.

11. Marker element (1) according to Claim 9 or 10, **characterized in that** the sleeve body (15) has widenings (16) at its circumferential ends to prevent the hollow cylindrical element (13) into which the sleeve body (15) is inserted becoming detached from the sleeve body (15).

12. Device for the delivery (5) of a self-expanding stent (2) into a body vessel, comprising
- an outer sheath (9) which holds a stent (2) under radial compression in a distal portion of the delivery system (5),
- a pusher (11) guided in the outer sheath (9) with its distal end pushing against the proximal end of the stent (2),
- a tubular element (6) which passes through the compressed stent (2) and the pusher (11),
**characterized in that** it also has a marker element (1) which is arranged on the tubular element (6) such that it can slide freely backwards and forwards but cannot become detached and is connected to the radially compressed stent (2) in a detachable manner.

13. Delivery system (5) according to Claim 12, **characterized in that** the outer sheath (9) has an outer sheath marker (10) at its distal end.

14. Delivery system (5) according to Claim 12 or 13, **characterized in that** the tubular element is a guide wire catheter (6).

15. Delivery system (5) according to one of Claims 12 to 14, **characterized in that** it is fitted with a marker element according to one of Claims 1 to 10.

## Revendications

1. Marqueur (1) destiné à marquer une ou plusieurs délimitations d'un segment d'un stent (2) auto-expansible présentant des mailles (20), le marqueur (1) présentant un matériau radio-opaque et pouvant être placé sur un dispositif (5) d'introduction du stent (2) auto-expansible dans un vaisseau corporel, le dispositif (5) présentant un élément (6) tubulaire qui est introduit dans le stent (2) et le marqueur (1) étant en prise de façon détachable dans les maille (20) du stent (2), **caractérisé en ce que** le marqueur (1) peut être monté entre le stent (2) et l'élément tubulaire (6) de façon à pouvoir être décalé sur l'élément tubulaire (6).

2. Marqueur (1) selon la revendication 1, **caractérisé en ce que** l'élément tubulaire est un cathéter de guidage d'un fil (6)

3. Marqueur (1) selon la revendication 1 ou 2, **caractérisé en ce qu'**il se détache du stent (2) lors de la libération et de l'expansion du stent (2).

4. Marqueur (1) selon l'une des revendications 1 à 3, **caractérisé en ce qu'**il marque une extrémité d'une zone fonctionnelle (3) du stent (2), la zone fonctionnelle (3) constituant un segment du stent (2) qui présente un angle de tressage inférieur à celui aux autres segments adjacents du stent (2) dans le sens longitudinal.

5. Marqueur (1) selon l'une des revendications 1 à 4, **caractérisé en ce qu'**il présente un élément formant un cylindre creux (13) qui est lui-même en prise avec au moins une proéminence (14), de préférence, 1 à 12, dirigée radialement vers l'extérieur, qui est en prise avec les mailles (20) du stent (2).

6. Marqueur (1) selon la revendication 5, **caractérisé en ce qu'**au moins une proéminence (14) a la forme d'un prisme.

7. Marqueur (1) selon la revendication 5, **caractérisée en ce qu'**au moins une proéminence (14) a une forme pyramidale.

8. Marqueur (1) selon l'une des revendications 1 à 7, **caractérisé en ce qu'**il est complètement constitué d'un matériau radio-opaque.

9. Marqueur (1) selon l'une des revendications 5 à 7, **caractérisé en ce** qui présente en outre une gaine (15) qui est insérée dans l'élément formant un cylindre creux (13) et qu'il est reliée fixement à celui-ci.

10. Marqueur (1) selon la revendication 9, **caractérisé en ce que** la gaine (15) présente un matériau radio-opaque et l'élément formant un cylindre creux (13) présente un matériau non radio-opaque.

11. Marqueur selon la revendication 9 ou 10, **caractérisé en ce que** la gaine (15) présente à ses extrémités circonférentielles, des évasements (16) pour empêcher que l'élément formant un cylindre creux (13) dans lequel la gaine (15) est introduite, ne se détache de la gaine (15).

12. Dispositif d'introduction (5) d'un stent auto-expansible (2) dans un vaisseau corporel, présentant
- un tube d'enveloppe (9) qui maintient comprimé radialement un stent (2) dans un segment distal du système d'introduction (5),
- un élément pousseur (11) conduit dans le tube d'enveloppe (9) qui pousse avec son extrémité distale, sur l'extrémité proximale du stent (2)
- un élément tubulaire (6) qui est conduit dans le stent comprimé (2) et l'élément pousseur (11),
**caractérisé en ce qu'**il présente en outre un marqueur (1) qui peut être librement poussé dans un sens ou dans l'autre mais qui n'est pas amovible, sur lequel l'élément tubulaire (6) est monté et qui est relié de façon détachable avec le stent (2) comprimé radialement.

13. Système d'introduction (5) selon la revendication 12, **caractérisé en ce que** le tube d'enveloppe (9) présente un marqueur de tube d'enveloppe (10) à son extrémité distale.

14. Système d'introduction (5) selon la revendication 12 ou 13, **caractérisé en ce que** l'élément tubulaire est un cathéter de guidage de fil (6).

15. Système d'introduction (5) selon l'une des revendications 12 à 14, **caractérisé en ce qu'**il présente un marqueur selon l'une des revendications 1 à 10.
